# EUROPEAN PATENT SPECIFICATION

(11) **EP 1 105 392 B1**
(45) Date of publication and mention of the grant of the patent: **04.09.2002**
(21) Application number: 00944685.7
(22) Date of filing: 15.06.2000
(51) Int. Cl.: C07D 487/04, C07D 487/00

(54) **PROCESS FOR THE PREPARATION OF IMIDAZODIAZEPINE INTERMEDIATES**
PROZESS ZUR HERSTELLUNG VON ZWISCHEBPRODUKTEN FÜR IMIDAZODEAZEPINEN
PREPARATION D'INTERMEDIAIRES D'IMIDAZODIAZEPINE

(30) Priority: 30.06.1999 US 343317
(43) Date of publication of application: 13.06.2001
(60) Divisional application: 01130894.7
(73) Proprietor: ABBOTT LABORATORIES, Abbott Park, IL 60064-6050 (US)
(72) Inventor: DHAON, Madhup, K., Mundelein, IL 60060 (US); LABIB, Parvis, Gurnee, IL 60031 (US); DAVIS, Deborah, A., Gurnee, IL 60031 (US); ESSER, Grant, L., Des Plaines, IL 60018 (US)
(74) Representative: Modiano, Guido, Dr.-Ing.
(86) International application number: US0016476
(87) International publication number: WO01000625

(56) References cited:
- US-A- 4 307 237
- US-A- 4 440 685
- GILMAN N W ET AL: "Atropisomers of 1,4-benzodiazepines. 2. Synthesis and Resolution of Imidazo (1,5-a)(1,4)Benzodiazepines" THE JOURNAL OF ORGANIC CHEMISTRY, [Online] vol. 58, no. 12, 23 April 1993 (1993-04-23), pages 3285-3298, XP002901311
- WALSER A ET AL: "Quinazolines and 1,4-Benzodiazepines. XCV (1). Synthesis of 1,4-Benzodiazepines by Ring Expansion of 2-Chloromethylquinazolines with Carbanions" JOURNAL OF HETEROCYCLIC CHEMISTRY, vol. 23, no. 4, September 1986 (1986-09) - October 1986 (1986-10), pages 1303-1314, XP002901312

## Description

### Technical Field

The present invention relates to a process for the preparation of 8-chloro-6-(2-fluorophenyl)-1-methyl-4*H*-imidazo[1,5-*a*][1,4]benzodiazepine-3-carboxylic acid.

### Background of The Invention

Midazolam (8-chloro-6-(2-fluorophenyl)-1-methyl-4*H*-imidazo[1,5-*a*][l,4]benzodiazepine), a pre-operative anesthetic, belongs to a class of imidazobenzodiazepine compounds which are useful as anticonvulsants, sedatives, and muscle relaxants.

Synthesis of Midazolam has been described in US 4307237. A key step in this synthesis is the construction of the imidazole ring by conversion of methyl 7-chloro-5-(2-flourophenyl)-α-(hydroxyimino)-3H-1,4-benzodiazepine-2-acetate to 8-chloro-6-(2-fluorophenyl)-1-methyl-4*H*-imidazo[1,5-*a*][1,4]benzodiazepine-3-carboxylic acid. This conversion is effected via a three step process that requires isolation of the intermediates and column chromatography for the penultimate ester.

The large scale production of commercial drugs requires devising chemical syntheses that avoid complicating factors such as use of high cost reagents, chemicals that require special handling, lengthy multi-step synthetic sequences, chromatography of intermediates, and low-yielding steps. An effective strategy to lower the cost associated with multi-step processes is the reduction in the number of steps required to complete the synthesis by combining several steps into a "single pot" transformation. However, running multiple steps in a single reaction vessel or without purification of intermediates poses a challenge due to competing side reactions, solvent incompatibilities, and purification difficulties.

The present invention discloses a novel synthesis of 8-chloro-6-(2-fluorophenyl)-1-methyl-4*H*-imidazo[1,5-*a*][1,4]benzodiazepine-3-carboxylic acid that allows multiple reaction steps in a single reaction vessel without isolation of intermediates. In addition, this invention provides a process that avoids costly chromatography of the intermediates or product.

### Summary of The Invention

In one embodiment, the present invention discloses a process for preparing a compound having formula **I,** 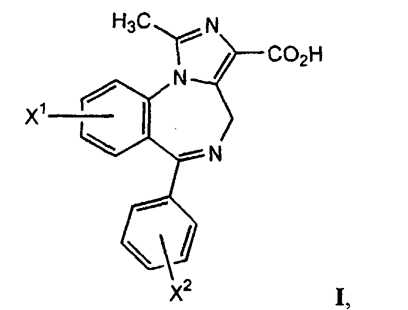 wherein X¹ and X² are independently selected from the group consisting of halogen, nitro, and amino comprising reacting a compound having formula **II,** 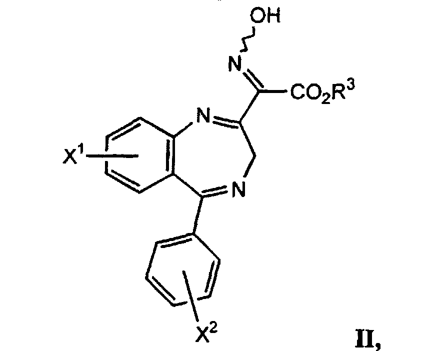 wherein R³ is hydrogen or alkyl and X¹ and X² are independently selected from the group consisting of halogen, nitro, and amino in a mixture comprising hydrogen, hydrogenation catalyst, trialkylorthoacetate or triarylorthoacetate, and an acid followed by removal of the catalyst and reaction with an alkali metal hydroxide to produce a compound of formula **I**.

In another embodiment, the present invention discloses a process for preparing a compound having formula **III,** 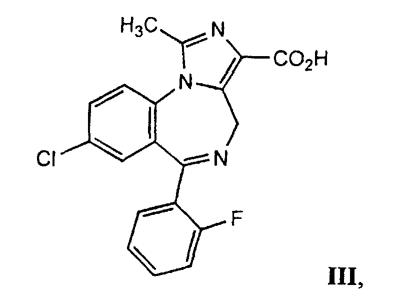 comprising reacting a compound having formula **IV,** 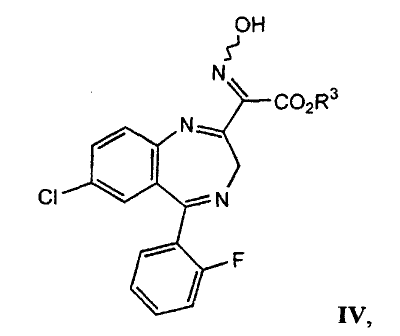 wherein R³ is an alkyl group in a mixture comprising hydrogen, hydrogenation catalyst, trialkylorthoacetate or triarylorthoacetate, and an acid followed by removal of the catalyst and reaction with an alkali metal hydroxide to produce a compound of formula **I.**

In yet another embodiment, the present invention discloses a process for preparing a compound having formula **III,** 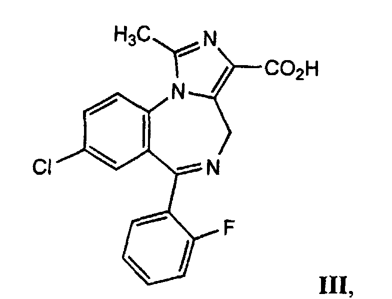 comprising reacting a compound having formula **IV**, 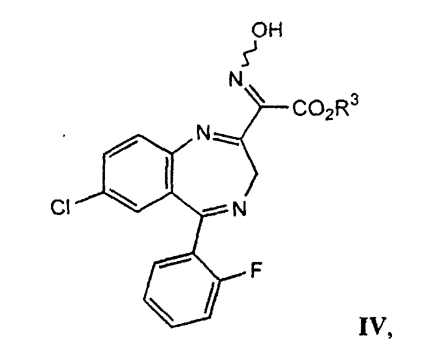 wherein R³ is an alkyl group in a mixture comprising hydrogen, Raney nickel, trimethylorthoacetate, and *para*-toluenesulfonic acid followed by removal of the Raney nickel catalyst and reaction with potassium hydroxide to produce a compound of formula **III.**

### Detailed Description of The Invention

All patents, patent applications, and literature references cited in the specification are hereby incorporated by reference in their entirety. In the case of inconsistencies, the present disclosure, including definitions, will prevail.

As used in the specification and the claims, the following terms have the meanings specified.

The term "alcoholic solvent," as used herein, refers to R⁸OH, wherein R⁸ is an alkyl group, as defined herein. Representative alcoholic solvents include methanol, ethanol, *iso*-propanol, *n*-propanol, *n*-butanol, *sec*-butanol, and the like.

The term "alkali metal ion," as used herein, refers to an ion derived from a metal selected from the group consisting of lithium, sodium, potassium, rubidium and cesium, and the like.

The term "alkali metal alkoxide," as used herein, refers to M-OR⁸, wherein M represents an alkali metal ion as defined herein and R⁸ represents an alkyl group as defined herein. Representative alkali metal alkoxides include potassium *tert*-butoxide, sodium ethoxide, and sodium *tert*-butoxide, and the like.

The term " alkoxide," as used herein, refers to a species having the formula ^{^{⊖}}O―R⁸,wherein R⁸ represents an alkyl group as defined herein, and ^{^{⊖}} represents a single negative charge. Representative alkoxides include *tert*-butoxide and ethoxide, and the like.

The term "alkyl," as used herein, refers to a straight or branched chain hydrocarbon radical having from one to twelve carbon atoms. Representative alkyl groups include methyl, ethyl, *n*-propyl, *iso*-propyl, 2-methylpropyl, *n*-butyl, 2-butyl, *tert*-butyl, *n*-pentyl, 1-methylbutyl, 2,2-dimethylbutyl, 2-methylpentyl, 2,2-dimethylpropyl, *n*-hexyl, and the like.

The term "amino," as used herein, refers to -NH₂.

The term '"aryl," as used herein, refers to a carbocyclic ring system having 6-10 ring atoms and one or two aromatic rings. Representative examples of aryl groups include phenyl, naphthyl, tetrahydronaphthyl, indanyl, indenyl, and the like.

The term "dialkyl halophosphate," as used herein, refers to X-P(=O)(-OR⁹)₂, wherein X is a halogen as defined herein, and R⁹ is an alkyl group. Representative dialkyl halophosphates include dimethyl chlorophosphate and diethyl chlorophosphate, and the like.

The term "diaryl halophosphate," as used herein, refers to X-P(=O)(-OR¹⁰)₂, wherein X is a halogen as defined herein, and R¹⁰ is an aryl group as defined herein. Representative diaryl halophosphates include diphenyl chlorophosphate, and the like.

The term "halogen," as used herein, refers to -Cl, -Br, and -I.

The term "hydrogenation catalyst," as used herein, refers to a substance that facilitates hydrogenation. Hydrogenation catalysts include nickel, palladium, platinum, rhodium, rhenium, copper, and iridium and compounds derived therefrom. Representative hydrogenation catalysts include Raney nickel and palladium on carbon, and the like.

The term " hydroxy" or "hydroxyl," as used herein, refers to -OH.

The term " mineral acid," as used herein, refers to an acid that does not contain carbon. Representative mineral acids include hydrochloric, sulfuric, nitric, and phosphoric acid, and the like.

The term "nitro," as used herein, refers to -NO₂.

The term " organic acid," as used herein, refers to an acid that contains carbon. Representative organic acids include acetic and para-toluenesulfonic acid, and the like.

The term "pharmaceutically acceptable salt," as used herein, refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well-known in the art. For example, S. M Berge, *et al.* describe pharmaceutically acceptable salts in detail in *J. Pharmaceutical Sciences,* **1977,** *66*:1-19. The salts can be prepared *in situ* during the final isolation of Midazolam, or separately by reacting the free base function with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalatc, propionate, stearate, succinate, sulfate, tartrate, thiocyanate, toluenesulfonate, undecanoate, valerate salts, and the like.

The term "psi," as used herein, refers to pounds-per-square-inch.

Representative alkali or alkaline earth metal cations include sodium, lithium, potassium, calcium, magnesium, and the like, as well as nontoxic ammonium, quaternary ammonium, and amine cations including, but not limited to, ammonium, tetramethylammonium, and tetraethylammonium, and the like.

The term "trialkylorthoacetate," as used herein, refers to CH₃C(-OR¹¹)₃, wherein R¹¹is an alkyl group.

The term "triarylorthoacetate," as used herein, refers to CH₃C(-OR¹²)₃, wherein R¹² is an aryl group.

The present invention contemplates geometric isomers and mixtures thereof. The symbol " 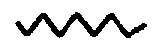 " indicates a single isomer or a mixture of isomers. For example, 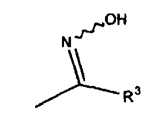 denotes a single isomeric oxime or a mixture of regioisomeric oximes where the hydroxyl can be disposed on the same side as R³ or on the opposite side of R³.

### Synthetic Methods

The compounds and processes of the present invention will be better understood in connection with the following synthetic scheme which illustrate the method by which the compounds of the invention may be prepared.

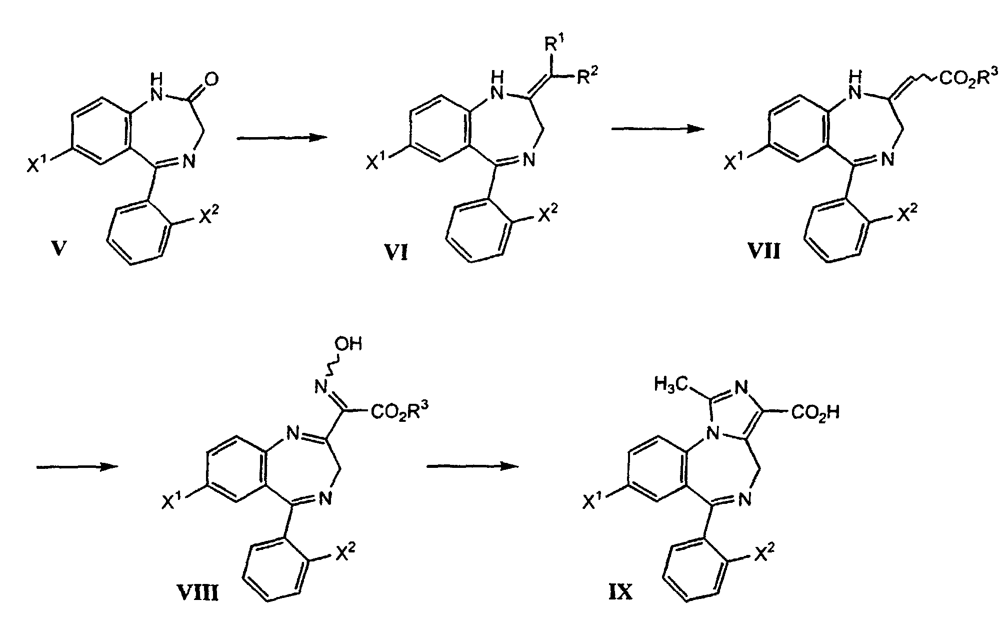

Halobenzodiazepine **V** (for example, X¹ is chlorine and X² is fluorine) was converted to vinyl compound **VI** by the following reaction sequence: 1) a dialkyl malonate or, alternatively, an alkyl cyanoacetate or other doubly activated methylene compound, was reacted with an alkali metal alkoxide such as potassium *tert*-butoxide in a solvent system that contains a mixture of hydrocarbon and polar solvents such as heptane/acetonitrile to produce the malonate anion; 2) the malonate anion was reacted with a dialkyl halophosphate such as, for example, diethyl chlorophosphate to form the phosphate anion; and 4) the phosphate anion was reacted with **V** to give **VI** (R¹ and R² are independently selected from the group comprising -CN and -CO₂R³, wherein R³ is alkyl). Vinyl compound **VI** was reacted with an alkali metal hydroxide such as, for example, potassium hydroxide in a suitable alcohol solvent at a temperature from about 45 °C to a about 100 °C to give α,β-unsaturated ester **VII** (for example, R³ is methyl). Ester **VII** was reacted with an alkali metal nitrite such as sodium nitrite and an acid such as acetic acid to give oxime **VIII.** The oxime was converted to **IX** in a single reaction vessel by the following reaction sequence: 1) oxime **VIII** was reacted with a mixture of hydrogen at a pressure of between 15-45 psi, a trialkylorthoacetate such as triethylorthoacetate, and a hydrogenation catalyst such as Raney nickel in a polar solvent system such as THF/methanol; 2) the catalyst was removed by filtration; and 3) the resulting mixture was reacted with an alkali metal hydroxide such as potassium hydroxide dissolved in a polar solvent such as water at a temperature from about 20 °C to about 40 °C to give **IX**.

The compounds and processes of the present invention will be better understood in connection with the following examples, which are intended as an illustration of and not a limitation upon the scope of the invention.

### Example 1

### Methyl 8-chloro-5-(2-flourophenyl)-α-(hydroxyimino)-3H-1,4-benzodiazepine-2-acetate

### Example 1a

Potassium *tert*-butoxide (51 g) in a mixture of acetonitrile (60 g) and heptane (240 g) was stirred for 15 minutes, then cooled to 5°C under a nitrogen atmosphere. A solution of diethyl malonate (71 g) in acetonitrile (90 g) was added over 30 minutes. To the resulting suspension was added diethyl chlorophosphate (26 g) in acetonitrile (30 g). After agitation for 1 hour, 7-chloro-5-(2-fluorophenyl)-1,3-dihydro-2*H*-1,4-benzodiazepin-2-one (desalkylflurazepam) (21 g) was added in portions. The resulting reaction mixture was stirred at room temperature for 16 hours, cooled to 10°C, and then decomposed by the addition of water (160 mL). The pH of the solution was adjusted to 5.0-5.6 with dilute hydrochloric acid, mixed for 1 hour, and filtered. The solid material obtained was washed with water (300 g) and heptane (100 g) and dried on the filter by applying a stream of nitrogen to give example 1a.

### Example 1b

Example 1a was charged back to the reaction flask, and methanol (250 g) and potassium hydroxide (5 g) were added. The suspension was heated to reflux under nitrogen for 5 hours, cooled to 5°C, and agitated for 1 hour. The solid material obtained was filtered, and the filter cake was washed with methanol (45 g) and dried under nitrogen to yield example 1b.

### Example 1c

Example 1b was dissolved in acetic acid (165 g) at room temperature, and sodium nitrite (15 g) was added in portions. The reaction mixture was mixed for 2 hours and filtered. The filter cake was washed with water (100 g), toluene (50 g), and methanol (60 g). The solids were suspended in methanol (160 g), heated to reflux for 4 hours, cooled to room temperature, and filtered. The filter cake was washed with methanol (50 g) and dried under nitrogen to yield 16.8 g of methyl 8-chloro-5-(2-flourophenyl)-α-(hydroxyimino)-3H-1,4-benzodiazepine-2-acetate (example 1c).

### Example 2

### 8-Chloro-6-(2-flourophenyl)-1-methyl-4H-imidazo[1,5-a][1,4]benzodiazepine3-carboxylic acid (tricyclic acid)

Raney nickel (18.7 g) was washed with methanol and transferred to a hydrogenation vessel. To this was added methanol (94 g), example 1c (18.7 g),*para*-toluenesulfonic acid (2.9 g), triethylorthoacetate (57.0 g), and THF (168 g). The reaction mixture was hydrogenated at 30 psi for 16 hours and filtered under nitrogen. The Raney nickel cake was washed with methanol, and a cooled solution of potassium hydroxide (22 g in 100 g water) was added in portions to the reaction solution. The temperature was kept below 30°C, and the reaction solution was stirred for 2 hours. The solvent was distilled off under vacuum and water (125 g) was added. The aqueous solution was washed with isopropylacetate (3 X 125 g). The aqueous phase was adjusted to a pH of 5.6-6.1 with glacial acetic acid with vigorous stirring. The product that separated out was filtered, washed with water (50 g) and then heptane (100 g), and dried on the filter. Purification was carried out by heating a mixture of isopropyl alcohol/heptane and the product to reflux, filtering, and drying to give 10 g of the tricyclic acid.
mp 270 -273 °C (lit. 271 °C-274 °C);
MS (M+H)+ m/e 370.

## Claims

1. A process for preparing a compound having formula **I,** 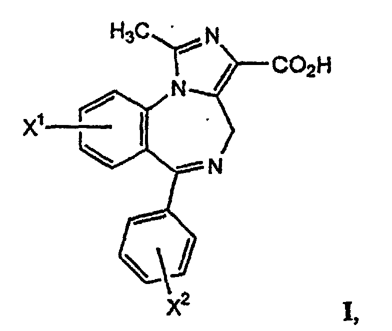 wherein X¹ and X² are independently selected from the group consisting of halogen, nitro, and amino;
said process comprising reacting a compound having formula **II**. 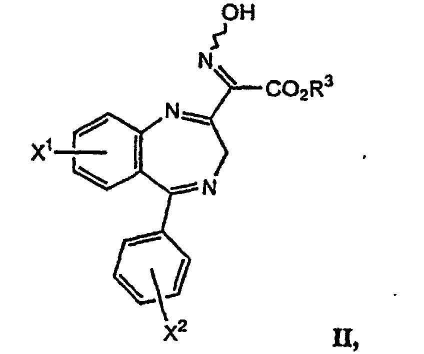 wherein R³ is hydrogen or alkyl and X¹ and X² are independently selected from the group consisting of halogen, nitro, and amino in a mixture comprising hydrogen, hydrogenation catalyst, trialkylorthoacetate or triarylorthoacetate, and an acid followed by removal of the catalyst and reaction with an alkali metal hydroxide to produce a compound of formula **I.**

2. The process according to claim 1, wherein compound **I** has the following formula: 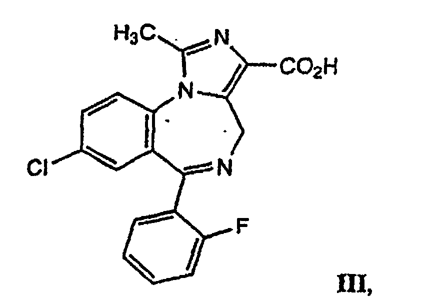 and compound **II** has the following formula: 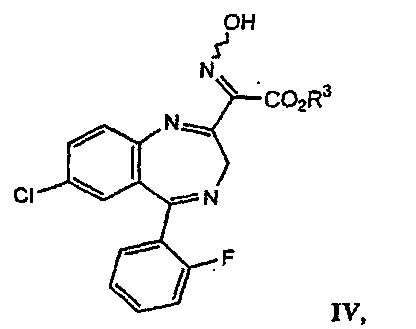 wherein R³ is an alkyl group.

3. The process according to claim 2, wherein the catalyst is a hydrogenation catalyst.

4. The process according to claim 3, wherein the catalyst is Raney nickel.

5. The process according to claim 2, wherein the trialkylarthoacetate is selected from the group consisting of triethylorthoacetate and trimethylorthoacetate.

6. The process according to claim 5, wherein the trialkylorthoacetate is triethylorthoacetate.

7. The process according to claim 2, wherein the acid is selected from the group consisting of a mineral acid and an organic acid.

8. The process according to claim 7, wherein the acid is selected from the group consisting of *para*-toluenesulfonic acid, acetic acid, hydrochloric acid, sulfuric acid, nitric acid and phosphoric acid.

9. The process according to claim 8, wherein the acid is *para*-toluenesulfonic acid.

10. The process according to claim 2, wherein the alkali metal ion of the alkali metal hydroxide is selected from the group consisting of lithium, sodium, and potassium.

11. The process according to claim 10, wherein the alkali metal hydroxide is potassium or sodium hydroxide.

12. The process according to claim 11, wherein the alkali metal hydroxide is potassium hydroxide.

13. The process according to claim 2 wherein compound III is produced without isolation or purification of intermediates.

## Patentansprüche

1. Ein Verfahren zur Herstellung einer Verbindung mit der Formel **I**, 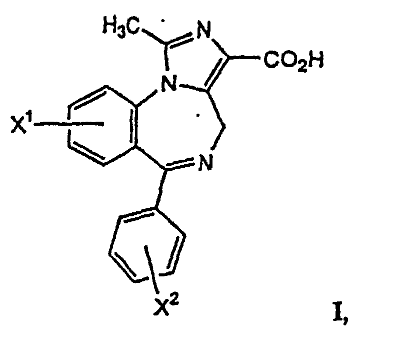 worin X¹ und X² unabhängig gewählt sind aus der Gruppe bestehend aus Halogen, Nitro und Amino;
wobei das Verfahren die Reaktion einer Verbindung mit der Formel **II** umfaßt, 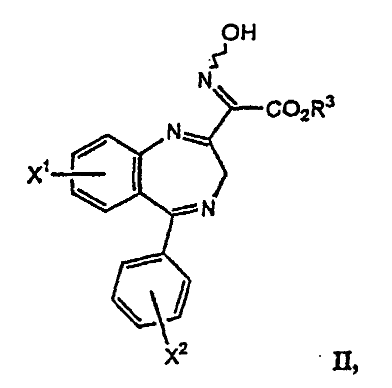 worin R³ Wasserstoff oder Alkyl ist, und X¹ und X² unabhängig gewählt sind aus der Gruppe bestehend aus Halogen, Nitro und Amino, in einer Mischung, die Wasserstoff, einen Hydrierungskatalysator, Trialkylorthoacetat oder Triarylorthoacetat und eine Säure umfaßt, gefolgt von der Entfernung des Katalysators und der Reaktion mit einem Alkalimetallhydroxid zur Herstellung einer Verbindung der Formel **I**.

2. Das Verfahren gemäß Anspruch 1, wobei die Verbindung **I** folgende Formel hat: 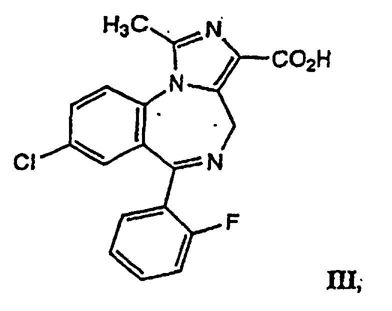 und Verbindung **II** hat folgende Formel: 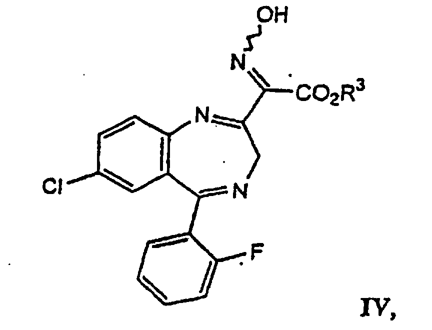 worin R³ eine Alkylgruppe ist.

3. Das Verfahren gemäß Anspruch 2, worin der Katalysator ein Hydrierungskatalysator ist.

4. Das Verfahren gemäß Anspruch 3, worin der Katalysator Raney-Nickel ist.

5. Das Verfahren gemäß Anspruch 2, worin das Trialkylorthoacetat gewählt ist aus der Gruppe bestehend aus Triethylorthoacetat und Trimethylorthoacetat.

6. Das Verfahren gemäß Anspruch 5, worin das Trialkylorthoacetat Triethylorthoacetat ist.

7. Das Verfahren gemäß Anspruch 2, worin die Säure gewählt ist aus der Gruppe bestehend aus einer Mineralsäure und einer organischen Säure.

8. Das Verfahren gemäß Anspruch 7, worin die Säure gewählt ist aus der Gruppe bestehend aus *para*-Toluensulfonsäure, Essigsäure, Salzsäure, Schwefelsäure, Salpetersäure und Phosphorsäure.

9. Das Verfahren gemäß Anspruch 8, worin die Säure *para*-Toluensulfonsäure ist.

10. Das Verfahren gemäß Anspruch 2, worin das Alkalimetallion des Alkalimetallhydroxides gewählt ist aus der Gruppe bestehend aus Lithium, Natrium und Kalium.

11. Das Verfahren gemäß Anspruch 10, worin das Alkalimetallhydroxid Kalium- oder Natriumhydroxid ist.

12. Das Verfahren gemäß Anspruch 11, worin das Alkalimetallhydroxid Kaliumhydroxid ist.

13. Das Verfahren gemäß Anspruch 2, worin die Verbindung III ohne Isolierung oder Reinigung von Zwischenstoffen hergestellt wurde.

## Revendications

1. Procédé pour la préparation d'un composé ayant la formule **I**, 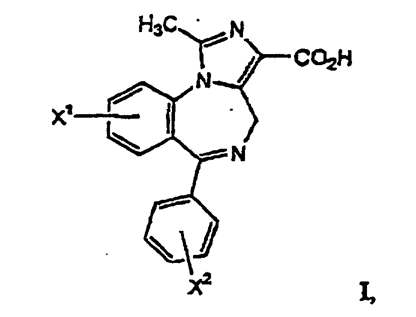 où X¹ et X² sont choisis de façon indépendante dans le groupe constitué par les atomes d'halogène et les groupes nitro et amino ;
ledit procédé comprenant la réaction d'un composé ayant la formule **II**. 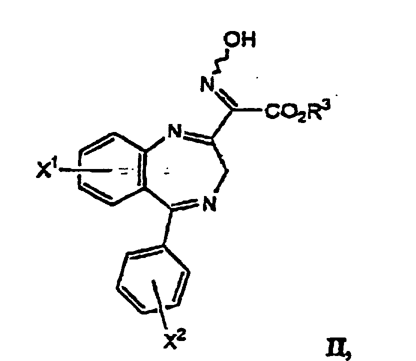 où R³ est un hydrogène ou un groupe alkyle et X¹ et X² sont choisis de façon indépendante dans le groupe constitué par les atomes d'halogène et les groupes nitro et amino, dans un mélange comprenant de l'hydrogène, un catalyseur d'hydrogénation, de l'orthoacétate de trialkyle ou de l'orthoacétate de triaryle, et un acide, réaction suivie de l'élimination du catalyseur et de la réaction avec un hydroxyde de métal alcalin pour produire un composé de formule I.

2. Procédé selon la revendication 1, dans lequel le composé **I** a la formule suivante : 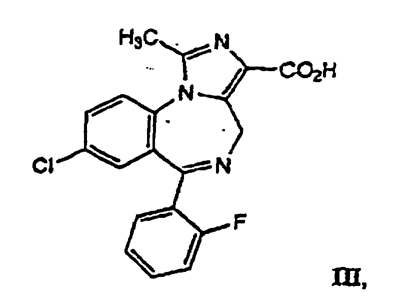 et le composé **II** a la formule suivante : 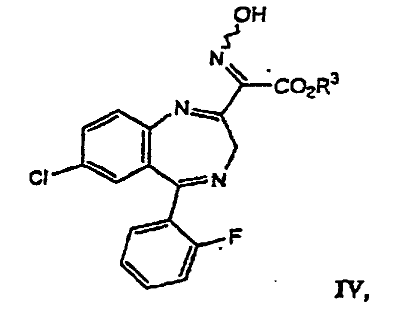 où R³ est un groupe alkyle.

3. Procédé selon la revendication 2, dans lequel le catalyseur est un catalyseur d'hydrogénation.

4. Procédé selon la revendication 3, dans lequel le catalyseur est du nickel de Raney.

5. Procédé selon la revendication 2, dans lequel l'orthoacétate de trialkyle est choisi dans le groupe constitué par l'orthoacétate de méthyle ou l'orthoacétate de triméthyle.

6. Procédé selon la revendication 5, dans lequel l'orthoacétate de trialkyle est l'orthoacétate de triéthyle.

7. Procédé selon la revendication 2, dans lequel l'acide est choisi dans le groupe constitué par un acide minéral et un acide organique.

8. Procédé selon la revendication 7, dans lequel l'acide est choisi dans le groupe constitué par l'acide para-toluènesulfonique, l'acide acétique, l'acide chlorhydrique, l'acide sulfurique, l'acide nitrique et l'acide phosphorique.

9. Procédé selon la revendication 8, dans lequel l'acide est l'acide para-toluènesulfonique.

10. Procédé selon la revendication 2, dans lequel l'ion métal alcalin de l'hydroxyde de métal alcalin est choisi dans le groupe constitué par le lithium, le sodium et le potassium.

11. Procédé selon la revendication 10, dans lequel l'hydroxyde de métal alcalin est l'hydroxyde de potassium ou de sodium.

12. Procédé selon la revendication 11, dans lequel l'hydroxyde de métal alcalin est l'hydroxyde de potassium.

13. Procédé selon la revendication 2, dans lequel le composé III est produit sans isolement ni purification des intermédiaires.
